# EUROPEAN PATENT APPLICATION

(11) **EP 1 820 490 A2**
(43) Date of publication of application: **22.08.2007**
(21) Application number: 07001589.6
(22) Date of filing: 25.01.2007
(51) Int. Cl.: A61K 8/41, A61K 8/73, A61K 8/81, A61Q 3/02

(54) **Custom-colored nail compositions**

(30) Priority: 13.02.2006 US 352600
(71) Applicant: L'Oréal, 75008 Paris (FR)
(72) Inventor: Weber, Robert, Suffern, NY 10901 (US); Patel, Nishith, Rosellen Park, NJ 07204 (US)
(74) Representative: Le Coupanec, Pascale A.M.P.

(57) **Abstract**

A process for making a custom-colored cosmetic nail composition involving the steps of:(a) providing a first and second gelled nail polish composition, each of which having a Brookfield viscosity of at least 20 poise, the compositions containing: (i) at least one gelling agent; (ii) at least one film forming polymer; (iii) at least one solvent; and (iv) at least one colorant present in at least one gelled nail polish composition; (b) providing at least one chemical viscosity reducing agent; and (c) combining the first and second gelled nail polish compositions, and the at least one chemical viscosity reducing agent, to form a custom-colored nail polish varnish having a Brookfield viscosity of at most 30 poise.

## Description

Nail polish varnishes are typically made available to consumers in pre-made colors, shades and styles. As a result, the likelihood of numerous consumers wearing an identically-colored nail varnish is quite high. In today's society, however, consumers value uniqueness, i.e., the ability to distinguish oneself from the rest. Conventional nail varnishes, because of their pre-formulation, provide little opportunity for customization of nail polish colors, shades and styles. Consequently, a large variety of colors and shades need to be made in order to offer numerous options to the consumer. As a result, transport and storage issues are problems for manufacturers, retailers and salons who need to make, deliver, and stock all of these colors/shades.

Moreover, because these products require the use of large amounts of volatiles, they need to be packaged in small glass containers which, by their very nature, are breakable, thus requiring special handling procedures. Thus, due to the packaging, retailers, professional salons and consumers need to exercise care in the handling and storage of nail polish varnishes.

A need, therefore, exists for a nail polish composition and system which easily facilitates customization of nail polish varnish color/appearance and which requires little, if any, special handling/storage procedures.

The present invention is directed to a process for making a custom-colored nail polish varnish comprising at least the steps of:(a) providing a first gelled nail polish composition having a Brookfield viscosity of at least 20 poise, the composition containing: (a)(i) at least one gelling agent; (a)(ii) at least one film forming polymer; (a)(iii) at least one solvent; and (a)(iv) at least one colorant; (b) providing a second gelled nail polish composition having a Brookfield viscosity of at least 20 poise, the composition containing: (b)(i) at least one gelling agent; (b)(ii) at least one film forming polymer; (b)(iii) at least one solvent; and (b)(iv) optionally, at least one colorant different from (a)(iv): (c) providing at least one chemical viscosity reducing agent; and (d) combining the first gelled nail polish composition, the second gelled nail polish composition, and the at least one chemical viscosity reducing agent to form a custom-colored nail polish varnish having a Brookfield viscosity of at most 30 poise.

According to another embodiment of the present invention, there is provided a process for making up nails comprising coating the nails with the above-described custom-colored nail polish composition. The process may be performed immediately prior to application of the nail polish vanish onto nails.

According to yet another embodiment of the present invention, there is provided a multi-unit kit for making custom-colored nail polish varnishes, the kit comprising: (a) at least one unit having a first gelled nail polish composition having a Brookfield viscosity of at least 20 poise, the composition containing: (a)(i) at least one gelling agent; (a)(ii) at least one film forming polymer; (a)(iii) at least one solvent; and (a)(iv) at least one colorant; (b) at least one unit having a second gelled nail polish composition having a Brookfield viscosity of at least 20 poise, the composition containing: (b)(i) at least one gelling agent; (b)(ii) at least one film forming polymer; (b)(iii) at least one solvent; and (b)(iv) optionally, at least one colorant different from (a)(iv); and (c) at least one unit containing at least one chemical reducing agent capable of reducing the viscosity of the first and/or second gelled nail polish composition.

Other than in the operating examples, or where otherwise indicated, all numbers expressing quantities of ingredients and/or reaction conditions are to be understood as being modified in all instances by the term "about".

All Brookfield viscosity values referenced herein are measured using a T-E or T-F spindle, at 6 rpm, at room temperature, for a period of 10 minutes.

Gelled Nail Polish Compositions

The gelled nail polish compositions of the present invention which are used to make the custom-colored nail polish varnish contain: (a) at least one gelling agent; (b) at least one film forming polymer; (c) at least one solvent; and (d) at least one colorant.

Gelling Agent

The gelled nail polish composition according to the invention comprises a gelling agent in a sufficient quantity to carry a pre-determined concentration of colorant. The gelling agent must be capable of exhibiting a drop in viscosity in response to a change in its environment such as, for example, chemical, mechanical or thermal input. Changes in polarity and concentration may also be employed in order to effectuate the drop in viscosity.

The gelling agent may be hydrophilic for an aqueous medium, or lipophilic for an oily medium.

Examples of suitable gelling agents for aqueous mediums include, but are not limited to, hydrophilic clays, and hydrophilic fumed silica.

Clays are silicates comprising a cation which may be chosen from calcium, magnesium, aluminum, sodium, potassium and lithium cations, and mixtures thereof.

Suitable hydrophilic clays may include, but are not limited to, clays of the family of smectites such as montmorillonites, hectorites, bentonites, beidellites and saponites, and of the family of vermiculites, stevensite, chlorites, and synthetic hectorites (also called laponites), bentonites, magnesium and aluminum, and calcium silicates, and their mixtures.

Suitable hydrophilic fumed silicas may include, but are not limited to, those commercially available under the names "AEROSIL^{®}.", by the company Degussa, and "CAB-O-SIL^{®}." by the company Cabot.

Examples of suitable gelling agents for the oily medium may be chosen from organophilic clays, hydrophobic fumed silicas, modified cellulose derivatives, and elastomeric organopolysiloxanes.

The organophilic clays are clays modified by chemical compounds making the clay capable of swelling in oily media.

Suitable organophilic clays may include, but are not limited to, montmorillonite, bentonite, hectorite, attapulgite, sepiolite, and mixtures thereof.

These clays may be modified with a chemical compound chosen from quaternary amines, tertiary amines, amine acetates, imidazolines, amine soaps, fatty sulphates, alkyl aryl sulphonates, amine oxides, and mixtures thereof.

Hydrophobic fumed silica may be obtained by chemically modifying the surface of the silica via a chemical reaction generating a reduction in the number of silanol groups and/or a substitution of silanol groups by hydrophobic groups.

Suitable hydrophobic silicas may include, but are not limited to, silicas treated with trimethylsiloxy, dimethylsilyloxy or polydimethylsiloxane groups.

Suitable modified cellulose derivatives may be chosen from those disclosed in US2005/0100518, the entire contents of which are hereby incorporated by reference.

The elastomeric organopolysiloxanes are in general partially or completely crosslinked and optionally have a three-dimensional structure.

Suitable elastomeric organopolysiloxanes may be obtained by addition and crosslinking reaction of at least:(a) one organopolysiloxane having at least two lower alkenyl groups per molecule; (b) one organopolysiloxane having at least two hydrogen atoms linked to a silicon atom per molecule; and(c) a platinum type catalyst.

Suitable elastomeric organopolysiloxanes may also include, but are not limited to, polyorganopolysiloxanes comprising R₂SiO and RSiO_{1.5} units and optionally R₃SiO_{0.5} and/or SiO₂ units wherein the radicals R, which may be identical or different, may be chosen from a hydrogen atom, a lower alkyl group, an aryl group, and an unsaturated aliphatic group such as a vinyl.

According to a preferred embodiment, the gelling agent is employed in an amount sufficient to carry the requisite amount of colorant necessary to enable single coat coverage of the nail polish varnish onto nails. The precise amount used depends on the color, shade and style of nail polish varnish being formulated and, as a result, is determined by those of ordinary skill in the art.

In general, the gelling agent may be present in the gelled nail polish composition in an amount of from 1 to 5% by weight, preferably from 1.5 to 4% by weight, preferably from 2 to 3.5% by weight, all weights based on the total weight of the composition.

In the event the gelling agent used is a thixotropic compound such as, for example, bentone, an activating compound should be employed. The gelling agent is in particular activated bentone. The activating compound is a compound which causes the thixotropic compound to swell, thereby allowing more colorant to be carried by the gelled nail polish composition. In the case of clays, the activating compound is generally an acid dissolved in a polar solvent such as isopropanol. Typically, the thixotropic compound may be activated by mixing it in a solvent and, preferably, in the presence of a film-forming polymer as well. While any solvent capable of dissolving the thixotropic agent and, if desired, the film-forming polymer, may be employed, a particularly preferred solvent is isopropanol.

In the event a thixotropic compound is used as the gelling agent, it can be employed in pre-activated form. An example thereof is stearalkonium hectorite commercially available from Elementis under the tradename Bentone 27 V.

Film-forming Polymer

The composition also contains at least one film-forming polymer. The term "film-forming polymer" is understood to mean a polymer capable of forming, alone and/or in the presence of a plasticizer, an isolable film. The film-forming polymer can be dissolved or dispersed in the form of particles in the solvent of the composition.

The film-forming polymer can be chosen from radical polymers, polycondensates and polymers of natural origin.

Suitable film-forming polymers include, but are not limited to, vinyl and acrylic polymers, polyurethanes, polyesters, alkyd resins, epoxy ester resins, cellulose polymers, such as nitrocellulose, cellulose esters, such as cellulose acetate, cellulose acetate propionate or cellulose acetate butyrate, resins resulting from the condensation of formaldehyde with an arylsulphonamide, and their mixtures.

When the composition comprises an aqueous medium, the film-forming polymer is generally present in the form of dispersed particles, thus forming a latex or pseudolatex.

Suitable film-forming polymers for use in an aqueous medium, include, but are not limited to, anionic polyurethanes, polyester-polyurethanes, polyether-polyurethanes, acrylic, acrylic/styrene, polyesters, alkyd resins, natural gums, and their mixtures.

Examples of commercially available acrylic dispersions include those sold under the tradenames Neocryl^{®} by Zeneca or Dow Latex 432^{®} by Dow Chemical. Examples of commercially available polyurethane dispersions are sold under the names "Avalure^{®}" or "Sancure^{®}" by Goodrich and "Neorez^{®}" by Avecia.

The film-forming polymer may be present in the gelled nail polish composition in an amount of from 0.5 to 20% by weight, preferably from 1 to 15% by weight, preferably from 5 to 12% by weight, based on the total weight of the composition.

Solvent

The composition according to the invention also comprises at least one solvent.

Suitable organic solvents are those which are liquid at ambient temperature and include, but are not limited to,:
- ketones such as methyl ethyl ketone, methyl isobutyl ketone, diisobutyl ketone, isophorone, cyclohexanone or acetone;
- alcohols, such as ethanol, isopropanol, diacetone alcohol, 2-butoxyethanol or cyclohexanol;
- glycols, such as ethylene glycol, propylene glycol, pentylene glycol or glycerol;
- propylene glycol ethers, such as propylene glycol monomethyl ether, propylene glycol monomethyl ether acetate or dipropylene glycol mono(n-butyl) ether;
- short-chain esters (having a total of 2 to 7 carbon atoms), such as ethyl acetate, methyl acetate, propyl acetate, n-butyl acetate or isopentyl acetate;
- alkanes, such as decane, heptane, dodecane or cyclohexane;
- aldehydes, such as benzaldehyde or acetaldehyde; and
- their mixtures.

The organic solvent is preferably chosen from short-chain esters (having a total of 2 to 8 carbon atoms), alcohols, and their mixtures.

The at least one solvent may also comprise an aqueous medium. In that case, suitable solvent candidates include, but are not limited to, water, water -miscible solvents, such as lower monoalcohols having from 1 to 5 carbon atoms, for example ethanol, glycols having from 2 to 8 carbon atoms, C₃-C₄ ketones and C₂-C₄ aldehydes, and combinations thereof.

The at least one solvent may be present in the gelled nail polish composition in an amount of from 10 to 80% by weight, preferably from 20 to 70% by weight, preferably from 30 to 60% by weight, based on the weight of the composition.

Colorant

At least one of the gelled nail polish compositions also comprises at least one colorant which can be chosen from pulverulent compounds and/or dyes which are soluble in the solvent of the composition.

The pulverulent compounds can be chosen from pigments and/or pearlescent agents and/or glitter generally used in nail varnishes.

The pigments can be white or colored and inorganic and/or organic. Mention may be made, among inorganic pigments, of titanium dioxide, which has optionally been surface-treated, zirconium, cerium, iron or chromium oxides, manganese violet, ultramarine blue, chromium hydrate, ferric blue or metal pigments, such as aluminum, copper or bronze. Mention may be made, among organic pigments, of carbon black, pigments of D & C type, lakes based on cochineal carmine or on barium, strontium, calcium or aluminum, or guanine.

Other pigments may also be chosen from pigments with an effect, such as particles comprising an organic or inorganic and natural or synthetic substrate, for example glass, acrylic, polyester, polyurethane or poly(ethylene terephthalate) resins, ceramics or aluminas, which substrate may or may not be covered with metal substances, such as aluminum, gold, copper or bronze, or with metal oxides, such as titanium dioxide, iron oxide or chromium oxide, or with inorganic or organic pigments, and their mixtures.

The pearlescent pigments may be chosen from white pearlescent pigments, such as mica covered with titanium oxide or with bismuth oxychloride, colored pearlescent pigments, such as titanium oxide-coated mica with iron oxides, titanium oxide-coated mica with in particular ferric blue or chromium oxide, or titanium oxide-coated mica with an organic pigment of the abovementioned type, and fluorophlogopites with iron oxides.

Other pigments may include those with goniochromatic properties, in particular liquid crystal or multilayer pigments.

The dyes are, for example, Sudan red, DC Red 17, DC Green 6, β-carotene, soybean oil, Sudan brown, DC Yellow 11, DC Violet 2, DC Orange 5 or quinoline yellow.

The colorant may also be chosen from fluorescent whitening agents.

In a preferred embodiment, the colorant(s) in particular (a)(iv) and (b)(iv) is/are employed in an amount sufficient to facilitate one coat coverage of the nail polish varnish onto a nail. The precise amount used depends on the color, shade and style of nail polish varnish being formulated and, as a result, will be determined by those of ordinary skill in the art.

In general, the colorant is present in at least one of the gelled nail polish compositions in an amount of from greater than 0 to 25% by weight, preferably from 1 to 20% by weight, preferably from 5 to 15% by weight, based on the total weight of the composition.

The gelled nail polish composition of the present invention will have a Brookfield viscosity of at least 20 poise, preferably at least 40 poise, preferably at least 60 poise.

Viscosity Reducing Agent

The chemical viscosity reducing agent of the present invention is used to reduce/break the viscosity of the above-described gelled nail polish compositions. Once the chemical viscosity reducing agent is combined with the gelled nail polish composition(s), the Brookfield viscosity, as defined above, of the resultant mixture, i.e., the custom-colored nail polish varnish, is at most 30 poise, preferably at most 20 poise, preferably at most 15 poise. In a particularly preferred embodiment, the viscosity of the custom-colored nail polish varnish is permanently reduced. However, it should be noted that so long as the viscosity of the custom-colored nail polish varnish is reduced for at least a period of time necessary for application of the varnish onto an individual's nails, its viscosity may subsequently increase, without departing from the spirit of the invention.

According to a preferred embodiment of the present invention, the at least one chemical viscosity reducing agent may be chosen from an alkaline compound an acidic compound and combinations thereof. Suitable alkaline compounds include, but are not limited to, metal hydroxides, amines, and the like.

According to one embodiment, the chemical viscosity reducing agent, preferably an alkaline compound, is employed in an amount sufficient to reduce the viscosity of the gelled nail polish composition(s). The viscosity will typically be reduced by at least 50% of its original viscosity, i.e., its viscosity immediately prior to introduction of the viscosity reducing agent, preferably by at least 60% of its original viscosity, and more preferably by at least 70% of its original viscosity.

The viscosity reducing agent should also be capable of rapidly reducing the viscosity of the gelled nail polish composition. Thus, once it is combined with the gelled nail polish composition, the resultant nail polish varnish should achieve a desired Brookfield viscosity within 4 minutes after combination with the gelled nail polish composition, preferably within 2 minutes, and more preferably within 1 minute.

In the event the viscosity reducing agent is an alkaline compound, it will typically be employed in an amount of from 0.05% to 0.20% by weight, preferably from 0.07 to 0.10% by weight, based on the weight of the composition.

Solvent capable of solubilizing the chemical compound

In order to be more effective in rapidly breaking the viscosity, it is preferred that the chemical compound, preferably an alkaline compound, be dissolved in a solvent, prior to its being combined with the above-described gelled nail polish composition. In the case of a gelled nail polish composition containing organic solvents, the chmical compound may be solubilized in a polar solvent compatible with the organic solvents. Suitable polar solvents may include, but are not limited to, ethanol, isopropanol, acetone, ethyl acetate, isobutyl acetate, butyl acetate, propyl acetate, isopropyl acetate, methyl ethyl ketone.

The solvent capable of solubilizing the chemical compound is employed in an amount sufficient to dissolve the chemical compound. Typically, this amount will range from 2 to 5% by weight, preferably from 2 to 4% by weight, preferably from 2.5 to 4% by weight, based on the weight of the composition.

It should be noted, however, that the solvent may be used in the absence of a chemical compound as the viscosity of the gelled nail polish composition may also be reduced through simple dilution. In that case, the solvent itself will serve as the viscosity reducing agent.

Auxiliaries

The composition according to the invention may additionally comprise an additional thickening agent different from the gelling agents described above.

When the gelled nail polish composition according to the invention comprises an aqueous medium, it may contain an additional thickening agent for an aqueous medium. This thickening agent is not capable, on its own, of giving the composition the thixotropic plastic character (nonthixotropic thickener); it makes it possible for example to adjust the viscosity of the composition in order to obtain a homogeneous flow.

When the gelled nail polish composition comprises an aqueous medium, the additional thickening agent may be chosen from hydrophilic thickening agents (nonthixotropic thickening agent for aqueous media). Suitable hydrophilic thickening agents may include, but are not limited to, water-soluble cellulosic thickeners, guar gums, quaternized guar gums, nonionic guar gums comprising C₁-C₆ hydroxyalkyl groups, xanthan, carob, scleroglucan, gellan, rhamsan and karaya gums, alginates, maltodextrin, starch and its derivatives, hyaluronic acid and its salts, polyglyceryl (meth)acrylate polymers, polyvinylpyrrolidones, polyvinyl alcohols, crosslinked polymers and copolymers of acrylamide, and associative polymers such as associative polyurethanes.

When the gelled nail polish composition comprises an oily medium, the additional thickening agent may be chosen from lipophilic thickening agents (nonthixotropic thickening agent for oily media).

Suitable additional lipophilic thickening agents may include, but are not limited to, alkylated guar gums, oil gelling polymers such as the polymers resulting from the polymerization or copolymerization of at least one monomer with an ethylenic group, and polyamide resins comprising alkyl groups having from 12 to 22 carbon atoms.

The additional thickening agent may be present in an amount of from 1 to 5% by weight, preferably from 1 to 4% by weight, all weights based on the total weight of the composition.

The composition may additionally comprise at least one plasticizer. Suitable plasticizers include, but are not limited to, glycols and their ether or ester derivatives, esters of acids, in particular carboxylic acids, such as citrates, adipates, carbonates, tartrates, phosphates or sebacates, oxyethylenated derivatives, such as oxyethylenated oils, and their mixtures.

The plasticizer may be present in the composition according to the invention in an amount of from 1 to 12% by weight, preferably from 3 to 8% by weight, all weights based on the total weight of the composition.

The gelled nail polish composition may additionally comprise any other additive or auxiliary commonly used in cosmetic compositions and known to a person skilled in the art as being capable of being incorporated in a nail varnish composition.

Such additives or auxiliaries may be chosen from coalescents, preservatives, fragrances, oils, waxes, surfactants, antioxidants, agents for combating free radicals, spreading agents, wetting agents, dispersing agents, antifoaming agents, neutralizing agents, stabilizing agents, active principles chosen from essential oils, UV screening agents, sunscreens, moisturizing agents, vitamins, proteins, ceramides, plant extracts, fibers, and the like, and their mixtures.

Of course, a person skilled in the art will take care to choose these possible additional compounds and/or their amounts so that the properties of the composition according to the invention are not, or not substantially, detrimentally affected by the envisaged addition.

According to one embodiment of the present invention, there is provided a process for making a custom-colored cosmetic nail composition involving the steps of:(a) providing a first gelled nail polish composition having a Brookfield viscosity of at least 20 poise, the composition containing: (a)(i) at least one gelling agent; (a) (ii) at least one film forming polymer; (a)(iii) at least one solvent; and (a)(iv) at least one colorant; (b) providing a second gelled nail polish composition having a Brookfield viscosity of at least 20 poise, the composition containing: (b)(i) at least one gelling agent; (b)(ii) at least one film forming polymer; (b)(iii) at least one solvent; and (b)(iv) optionally, at least one colorant different from (a)(iv); (c) providing at least one viscosity reducing agent; and (d) combining the first gelled nail polish composition, the second gelled nail polish composition, and the at least one viscosity reducing agent to form a custom-colored nail polish varnish having a Brookfield viscosity of at most 30 poise.

The combination of the gelled nail polish composition with the at least one chemical viscosity reducing agent will, preferably, involve a certain degree of physical mixing. Thus, while a chemical compound, such as an alkaline compound, is used as the viscosity reducing agent, its combination with the gelled nail polish composition will typically involve some input of mechanical energy. Means for combining the gelled nail polish composition with the chemical viscosity reducing agent may be provided. For example, a spatula may be used to physically mix the gelled nail polish composition and the chemical viscosity reducing agent in order to make the nail polish varnish. It should be noted, however, that any suitable means capable of generating a mechanical input of energy for blending/mixing may be employed, without departing from the spirit of the invention.

It should be noted that any number of gelled nail polish compositions varying to some degree in color/shade/style, etc. may be used in order to make the custom-colored nail polish varnish of the present invention.

In the event a chemical compound, such as an alkaline compound, is used as the viscosity reducing agent, the combination of the viscosity reducing agent with the gelled nail polish compositions will also require some degree of mechanical input of energy. For example, a spatula may be used to mix the gelled nail polish compositions and the viscosity reducing agent in order to form the custom-colored nail polish varnish. It should be noted, however, that any suitable mechanical means for blending/mixing may be employed, without departing from the spirit of the invention.

The blending/mixing of the viscosity reducing agent with the gelled nail polish compositions is preferably performed in a re-usable/washable container, such as, for example, one made of glass. Once the custom-colored nail polish varnish has been made, it may be applied onto nails in any conventional way such as, for example, using a brush.

According to yet another embodiment of the present invention, there is provided a multi-unit kit for making custom-colored nail polish varnishes. The kit comprises: (a) at least one unit having a first gelled nail polish composition as described above; (b) at least one unit having a second gelled nail polish composition as described above; and (c) at least one unit containing a chemical reducing agent capable of reducing the viscosity of the first and second gelled nail polish compositions.

The kit may additionally comprise a re-usable/washable container for blending/mixing the contents of the respective units, preferably a glass jar; mechanical means for blending/mixing and/or combining the ingredient contained in the respective units, preferably a small spatula; and means for applying the custom-colored nail polish varnish onto nails, preferably an artist brush for example a small artist brush. It should be understood, however, that any means suitable for effectuating the processes of the present invention may be employed without departing from the spirit of the present invention.

The units are preferably made of a flexible, non-breakable material such as, for example, plastic and may be configured in any desired shape such as, for example, a tube. It is because of this type of packaging, coupled with the highly pigmented nature of the gelled nail polish compositions, that conventional issues regarding handling and storage are, for the most part, a non-factor. The flexible containers are non-breakable and, therefore, much easier to handle and store.

It should be noted, however, that it is not necessary to use such packaging in order to effectuate the goal of being able to create custom-colored nail polishes per the process of the present invention.

Finally, because of the custom-blending facilitated by the present invention, fewer varieties of gelled nail polish compositions need to be carried by retailers and salons since an almost infinite number of colors, shades and styles can be custom-blended using a finite number of monochrome-type colors.

The present invention will be better understood from the examples which follow, all of which are intended for illustrative purposes only, and are not meant to unduly limit the scope of the invention in any way.

### Example 1: "Deep-Red" Shade Gelled Nail Polish Composition having a Brookfield viscosity of 3000 poise.

| INCI US | Concentration (%) |
|---|---|
| TRIBUTYL CITRATE | 7.97 |
| STEARALKONIUM HECTORITE | 2.49 |
| NITROCELLULOSE (and) ISOPROPYL ALCOHOL | 12.64 |
| ACRYLATES COPOLYMER¹ | 3.02 |
| PHTHALIC ANHYDRIDE/GLYCERIN/GLYCIDYL DECANOATE COPOLYMER | 14.03 |
| ETHYL ACETATE | 7.05 |
| BUTYL ACETATE | 43.72 |
| POLOXAMER 184 | 3.04 |
| PIGMENTS | 5.95 |
| POTASSIUM HYDROXIDE | 0.08 |
| TOTAL | 100.00% |

| | |
|---|---|
| ¹ Pecorez AC 50 | |

### Example 2: "Gold-Pearlescent" Shade Gelled Nail Polish

Composition having a Brookfield viscosity of 4000 poise.

| INCI US | Concentration (%) |
|---|---|
| TRIBUTYL CITRATE | 7.97 |
| STEARALKONIUM HECTORITE | 2.49 |
| NITROCELLULOSE (and) ISOPROPYL ALCOHOL | 12.64 |
| ACRYLATES COPOLYMER¹ | 3.02 |
| PHTHALIC ANHYDRIDE/GLYCERIN/GLYCIDYL DECANOATE COPOLYMER | 14.03 |
| ETHYL ACETATE | 7.05 |
| BUTYL ACETATE | 34.54 |
| POLOXAMER 184 | 3.04 |
| PERLESCENT PIGMENTS | 15.14 |
| POTASSIUM HYDROXIDE | 0.08 |
| TOTAL | 100.00% |

| | |
|---|---|
| ¹Pecorez AC 50 | |

A custom-blended nail polish varnish was prepared by dispensing approximately 1 ml. of the gelled nail polish compositions of Examples 1 and 2 into a single glass mixing jar, adding 3 drops of a 0.22 molar solution of potassium hydroxide to the mixing jar, and stirring the contents of the mixing jar with a spatula to form a custom-colored nail polish varnish in accordance with the present invention. The custom-colored nail polish varnish had a Brookfield viscosity of 20 poise.

## Claims

1. A process for making a custom-colored nail polish varnish comprising at least the steps of:
(a.) providing a first gelled nail polish composition having a Brookfield viscosity of at least about 20 poise, the composition containing: (a)(i) at least one gelling agent; (a) (ii) at least one film forming polymer; (a)(iii) at least one solvent; and (a)(iv) at least one colorant;
(b.) providing a second gelled nail polish composition having a Brookfield viscosity of at least about 20 poise, the composition containing: (b)(i) at least one gelling agent; (b)(ii) at least one film forming polymer; (b)(iii) at least one solvent; and (b)(iv) optionally, at least one colorant different from (a) (iv);
(c.) providing at least one chemical viscosity reducing agent; and
(d.) combining the first gelled nail polish composition, the second gelled nail polish composition, and the at least one chemical viscosity reducing agent to form a custom-colored nail polish varnish having a Brookfield viscosity of at most about 30 poise.

2. The process according to claim 1 wherein (a)(i) and (b)(i) are activated bentone.

3. The process according to claim 1 or 2 wherein the at least one viscosity reducing agent is chosen from an alkaline compound and an acidic compound.

4. The process according to any one of claims 1 to 3 further comprising providing means for combining the gelled nail polish composition with the chemical viscosity reducing agent.

5. The process according to anyone of claims 1 to 4 wherein the nail polish varnish achieves a Brookfield viscosity of at most about 30 poise within about 4 minutes after combination of the chemical viscosity reducing agent with the first and second gelled nail polish compositions.

6. The process according to any one of claims 1 to 5 wherein (a)(iv) and (b)(iv) are employed in an amount sufficient to facilitate one-coat coverage of the nail polish varnish onto a nail.

7. The process according to any one of claims 1 to 6 wherein the process is performed immediately prior to application of the nail polish varnish onto nails.

8. The product of the process according to any one of claims 1 to 7.

9. A process for making up nails comprising coating the nails with the nail polish varnish according to any one of claims 1 to 7.

10. A multi-unit kit for making custom-colored nail polish varnishes, the kit comprising:
(a.) at least one unit having a first gelled nail polish composition having a Brookfield viscosity of at least about 20 poise, the composition containing: (a)(i) at least one gelling agent; (a) (ii) at least one film forming polymer; (a)(iii) at least one solvent; and (a) (iv) at least one colorant;
(b.) at least one unit having a second gelled nail polish composition having a Brookfield viscosity of at least 20 poise, the composition containing: (b)(i) at least one gelling agent; (b)(ii) at least one film forming polymer; (b)(iii) at least one solvent; and (b)(iv) optionally, at least one colorant different from (a)(iv); and
(c.) at least one unit containing at least one chemical reducing agent capable of reducing the viscosity of the first and/or second gelled nail polish composition.

11. The kit according to claim 10 wherein (a)(i) and (b)(i) are activated bentone.

12. The kit according to claim 10 or 11 wherein the at least one chemical viscosity reducing agent is chosen from an alkaline compound and an acidic compound.

13. The kit according to any one of claims 10 or 12 further comprising means for combining the first and second gelled nail polish compositions with the chemical viscosity reducing agent.

14. The kit according to any one of claims 10 to 13wherein (a) (iv) and (b)(iv) are present in an amount sufficient to facilitate one-coat coverage of the nail polish varnish onto a nail.

15. The kit according to claim 13 wherein the means for combining the first and second gelled nail polish compositions with the chemical viscosity reducing agent is an artist brush.
